# EUROPEAN PATENT SPECIFICATION

(11) **EP 3 180 045 B1**
(45) Date of publication and mention of the grant of the patent: **19.02.2020**
(21) Application number: 15756277.8
(22) Date of filing: 13.08.2015
(51) Int. Cl.: A61L 31/00, A61L 31/14, A61B 17/08, A61B 17/064, A61B 17/12, A61B 17/82

(54) **TISSUE MATRICES AND METHODS OF TREATMENT**
GEWEBEMATRIZEN UND VERFAHREN ZUR BEHANDLUNG
MATRICES TISSULAIRES ET MÉTHODES DE TRAITEMENT

(30) Priority: 14.08.2014 US 201462037228 P; 15.05.2015 US 201562161987 P
(43) Date of publication of application: 21.06.2017
(73) Proprietor: LifeCell Corporation, Madison, New Jersey 07940 (US)
(72) Inventor: BACHRACH, Nathaniel, Clifton, NJ 07012 (US)
(74) Representative: Ashton, Timothy
(86) International application number: PCT/US2015/044971
(87) International publication number: WO 2016/025666

(56) References cited:
- WO-A1-03/068118
- WO-A1-2010/041101
- WO-A2-2007/131110
- US-A- 3 744 094
- US-A1- 2002 147 461

## Description

This application claims the benefit of co-pending, commonly assigned U.S. Provisional Patent Application No. 62/037,228, filed on August 14, 2014, and U.S. Provisional Patent Application No. 62/161,987, filed on May 15, 2015.

The present disclosure relates to products for tissue treatment as defined in the claims.

Various products are used as protective sleeves or anchors positioned around tissue or bone. Such products, however, can be difficult to position or secure around the tissue or bone when the position of the tissue or bone itself does not permit a slip over type of product to be placed thereon. As such, products wrapped around the tissue or bone are generally secured at opposing ends or to the tissue or bone with sutures.

In addition, sutures with or without various products are used to close incisions or to retain tissue or bone relative to other structures in the body of a patient. But the tension or position of such products and sutures generally cannot be easily adjusted without repositioning the sutures or products, and the use of sutures can also increase the amount of time necessary to secure tissue.

To cover tissue or bone and/or to close incisions or to retain tissue or bone relative to other structures in the body of a patient, it may be desirable to use compositions that have interlocking portions that facilitate attachment on or around adjacent tissue or bone without the use of sutures. Accordingly, methods of treatment including compositions, as well as compositions used in the methods, are provided.

US 2002/147461 describes a closure device consisting of a body defining a longitudinal axis and a plurality of tissue engaging elements on the body. The tissue engaging elements may be in the shape of annular flanges extending from the peripheral surface, and being expandable from a contracted condition towards an expanded condition to secure the closure device within a tissue opening. The closure device is formed from a biocompatible material, e.g. an extracellular matrix material as intestinal submucosa.

### SUMMARY

The scope of this invention is defined by the claims. Any references in the description to methods of treatment refer to the products of the present invention for use in a method for treatment of the human (or animal) body by therapy (or for diagnosis).

According to certain embodiments, a method of treatment is provided. The method can include providing a composition. The composition includes a composition body, at least a first component formed in the composition in the form of one or more holes or slits, and at least a second component formed in the composition body in the form of one or more tabs, the tabs having two opposing extensions. The first and second components are complementary relative to each other. The first and second components are configured to interlock relative to each other and/or soft tissue or bone. The composition includes at least a tissue matrix.

The method includes at least partially wrapping the composition around or positioning the composition onto the soft tissue, bone, or an implant. The method includes interlocking the first component relative to the second component or the soft tissue or bone to maintain the composition at least partially wrapped around or positioned on the tissue, bone, or implant.

In certain embodiments, the method includes forming one or more holes or slits in the body or host tissue of a patient and interlocking the second component with the host tissue by passing the second component through the hole or slit. In certain embodiments, the method includes interlocking two independent compositions relative to each other, soft tissue or bone, or both, by interlocking the respective first and second components of the compositions relative to each other.

In certain embodiments, the first and second components of the composition can be interlocked to form a three-dimensional construct, thereby creating, e.g., a tube or other shape, that can be used as a nerve conduit, a blood vessel, and the like.

The first component includes an opening passing through the composition body. The second component includes flared sections extending on opposing sides of the composition body. The method includes passing the flared sections through an opening in the composition body in a first direction. The flared sections can prevent passage of the flared sections through the opening in the composition body in a second direction opposing the first direction, thereby interlocking the first component relative to the second component.

More specifically, the first component includes one or more holes or slits passing through the composition body. The second component includes a tab including two opposing extensions along a length of the composition body. The method can include bending the two opposing extensions of the tab into a non-extending configuration and passing the two opposing extensions through a hole or slit in the composition body in a first direction. The method can include unbending the two opposing extensions after passage of the two opposing extensions through the hole or slit in the composition body. The two opposing extensions can prevent passage of the two opposing extensions through the hole or slit in the composition body in a second direction opposing the first direction, thereby interlocking the first component relative to the second component.

In certain embodiments, the tissue matrix is an acellular tissue matrix. In certain embodiments, the tissue matrix is a dermal tissue matrix.

In certain embodiments, interlocking the first component and the second component to the soft tissue or bone comprises passing a portion of soft tissue or bone through the first component and passing at least a portion of the second component through an incision or opening in the soft tissue or bone.

In certain embodiments, a composition for soft tissue or bone treatment is provided. The composition includes a composition body, at least a first component formed in the composition body, and at least a second component formed in the composition body. The composition body can be configured to be at least partially wrapped around or positioned onto soft tissue, bone, or an implant. The composition includes a tissue matrix. The first and second components are complementary relative to each other. The first and second components are configured to interlock relative to each other or the soft tissue or bone to maintain the composition at least partially wrapped around or positioned on the soft tissue, bone, or implant.

In certain embodiments, the tissue matrix is an acellular tissue matrix. In certain embodiments, the tissue matrix is a dermal tissue matrix. The composition body can be flexible to permit at least partially wrapping the composition body around the tissue, bone, or implant, and interlocking the first and second components relative to each other.

The first component includes one or more openings passing through the composition body. The second component includes flared sections extending on opposing sides of the composition body. The flared sections can be configured for passage through an opening in the composition body in a first direction. The flared sections can prevent passage of the flared sections through the opening in the composition body in a second direction opposing the first direction, thereby interlocking the first component relative to the second component.

More specifically, the second component includes a tab including two opposing extensions along a length of the composition body. The two opposing extensions can be configured to be bent into a non-extending configuration to pass the two opposing extensions through a hole or slit in the composition body in a first direction. The two opposing extensions can be configured to be unbent after passage of the two opposing extensions through the hole or slit in the composition body to prevent passage of the two opposing extensions through the hole or slit in the composition body in a second direction opposing the first direction, thereby interlocking the first component relative to the second component. The first and second components can be configured to remain interlocked relative to each other or the tissue or bone without the use of sutures.

According to certain aspects of the present disclosure, a method of treatment is provided. The method can include providing a composition. The composition includes a composition body and two components formed in the composition body. Each of the two components can be configured to interlock relative to tissue such as soft tissue or bone. The composition comprises a tissue matrix. The method includes at least partially wrapping the composition around or positioning the composition onto the tissue. The method includes interlocking each of the two components with the tissue to maintain the composition at least partially wrapped around or positioned on the tissue.

In certain aspects of the present disclosure, each of the two components includes flared sections extending on opposing sides of the composition body. The two components can extend from each other and be connected at a central portion of the composition body. The method includes passing the flared sections through an incision in the tissue in a first direction. The flared sections can be shaped to prevent passage of the flared sections through the incision in the tissue in a second direction opposing the first direction.

In certain aspects of the present disclosure, each of the two components includes a tab including two opposing extensions along a length of the composition body. The method includes bending the two opposing extensions of the tab into a non-extending configuration and passing the two opposing extensions through an incision in the tissue in a first direction. The method includes unbending the two opposing extensions after passage of the two opposing extensions through the incision in the tissue. The two opposing extensions can prevent passage of the two opposing extensions through the incision in the tissue in a second direction opposing the first direction.

In certain embodiments, the tissue matrix can be an acellular tissue matrix. In certain embodiments, the tissue matrix can be a dermal tissue matrix.

In certain aspects of the present disclosure, interlocking each of the two components with the tissue can form a cavity (e.g., a pocket) between the composition body and the tissue. The cavity can be configured and dimensioned to receive an implant therein.

According to certain embodiments, a product for tissue treatment is provided. The product comprises a body and two components formed in the body. The product can be configured to be at least partially wrapped around or positioned onto tissue. The product can comprise a tissue matrix. Each of the two components can be configured to interlock relative to the tissue to maintain the product at least partially wrapped around or positioned on the tissue.

In certain embodiments, the tissue matrix can be an acellular tissue matrix. In certain embodiments, the tissue matrix can be a dermal tissue matrix.

The composition body can be flexible to permit at least partially wrapping the composition body around the tissue and interlocking of the two components relative to the tissue. For example, the flexible composition body permits the composition to be shaped to conform with a part of the surface of the tissue.

In certain aspects of the present disclosure, each of the two components includes flared sections extending on opposing sides of the composition body. The flared sections can be configured for passage through an incision in the tissue in a first direction. In addition, the flared sections can be shaped to prevent passage of the flared sections through the incision in the tissue in a second direction opposing the first direction.

In certain embodiments, each of the components can include a tab including two opposing extensions along a length of the composition body. The two opposing extensions can be configured to be bent into a non-extending configuration to pass the two opposing extensions through an incision in the tissue in a first direction. The two opposing extensions can be configured to be unbent after passage of the two opposing extensions through the incision in the tissue to prevent passage of the two opposing extensions through the incision in the tissue in a second direction opposing the first direction. The components can thereby be configured to remain interlocked relative to the tissue without the use of suture.

According to certain aspects of the present disclosure, a method of treatment is provided. The method includes selecting a composition. The composition includes a composition body and a component, e.g., a tab, flared sections, or the like, formed in the composition body. The component can be configured to interlock with tissue and an anchoring material. The composition includes a tissue matrix.

The method includes selecting the anchoring material. The anchoring material can include an opening formed therein. The method includes at least partially passing the component through an opening formed in the tissue and the opening in the anchoring material. The method includes interlocking the component with the tissue and the anchoring material to maintain the anchoring material positioned adjacent to the tissue.

In certain aspects of the present disclosure, the method includes positioning the anchoring material adjacent to a wall of the tissue. The method includes aligning the opening of the anchoring material with the opening of the tissue.

In certain aspects of the present disclosure, the component can include flared sections extending on opposing sides of the composition body. At least partially passing the component through the openings in the tissue and the anchoring material can include passing the flared sections through the openings in the tissue and the anchoring material in a first direction. The flared sections can be shaped to prevent passage of the flared sections through the openings of the tissue and the anchoring material in a second direction opposing the first direction.

In certain aspects of the present disclosure, the component can include a tab including two opposing extensions. The method includes bending the two opposing extensions of the tab into a non-extended configuration and passing the two opposing extensions through the openings in the tissue and the anchoring material in a first direction. The method includes unbending the two opposing extensions after passage of the two opposing extensions through the openings in the tissue and the anchoring material. The two opposing extensions can prevent passage of the two opposing extensions through the openings in the tissue and the anchoring material in a second direction opposing the first direction.

In certain embodiments, the tissue matrix can be an acellular tissue matrix. In certain embodiments, the tissue matrix can be a dermal tissue matrix.

According to certain aspects of the present disclosure, a composition for tissue treatment is provided. The composition includes a composition body and a component, e.g., a tab, flared sections, or the like, formed in the composition body. The composition can be configured to interlock with tissue and an anchoring material. The component can be configured to be at least partially passed through openings formed in the tissue and the anchoring material. The composition comprises a tissue matrix. The component can be configured to interlock with the tissue and the anchoring material to maintain the anchoring material positioned adjacent to the tissue.

In certain embodiments, the tissue matrix can be an acellular tissue matrix. In certain embodiments, the tissue matrix can be a dermal tissue matrix.

In certain aspects of the present disclosure, the component can include flared sections extending on opposing sides of the composition body. The flared sections can be configured for passage through the openings in the tissue and the anchoring material in a first direction. The flared sections can be shaped to prevent passage of the flared sections through the openings in the tissue and the anchoring material in a second direction opposing the first direction.

In certain aspects of the present disclosure, the component can include a tab including two opposing extensions. The two opposing extensions can be configured to be bent into a non-extending configuration to pass the two opposing extensions through the openings in the tissue and the anchoring material in a first direction. The two opposing extensions can be configured to be unbent after passage of the two opposing extensions through the openings in the tissue and the anchoring material to prevent passage of the two opposing extensions through the openings in the tissue and the anchoring material in a second direction opposing the first direction. The component can thereby be configured to remain interlocked with the tissue and the anchoring material without the use of suture.

According to certain aspects of the present disclosure, a method of treatment is provided. The method includes providing a composition. The composition can include a first portion including a composition body and at least a first interlocking component formed in the composition body. The first interlocking component can be configured to interlock relative to tissue or an optional second portion of the composition. The composition comprises a tissue matrix. The method can include at least partially wrapping the first portion of the composition around or positioning the first portion of the composition onto an implant. The method includes interlocking the first interlocking component of the first portion with the tissue or the second portion to maintain the first portion of the composition at least partially wrapped around or positioned on the implant.

In certain aspects of the present disclosure, the first interlocking component includes a tab including two opposing extensions. In certain aspects of the present disclosure, the second portion of the composition includes at least one second interlocking component formed in the second portion. The second interlocking component can include an opening (e.g., one or more holes or slits) passing through the second portion. Interlocking the first interlocking component of the first portion with the tissue or the second portion can form a cavity (e.g., a pocket) configured to receive the implant.

The method can include bending the two opposing extensions of the tab into a non-extending configuration and passing the two opposing extensions through an opening in the tissue or the second portion in a first direction. The method can include unbending the two opposing extensions after passage of the two opposing extensions through the opening in the tissue or the second portion. The two opposing extensions can prevent passage of the two opposing extensions through the opening in the tissue or the second portion in a second direction opposing the first direction.

According to certain aspects of the present disclosure, a composition for tissue treatment is provided. The composition includes a first portion including a composition body. The composition includes at least a first interlocking component formed in the composition body. The first portion of the composition can be configured to be at least partially wrapped around or position onto an implant. The composition comprises a tissue matrix. The first interlocking component can be configured to interlock relative to tissue or a second portion of the composition to maintain the composition at least partially wrapped around or positioned on the implant.

The first interlocking component includes a tab including two opposing extensions. The second portion of the composition includes at least one second interlocking component formed in the second portion. The second interlocking component can include an opening (e.g., one or more holes or slits) passing through the second portion. The first portion of the composition can be configured to remain interlocked relative to the tissue or the second portion without the use of suture.

### DESCRIPTION OF THE DRAWINGS (figures 6-27 relate to reference embodiments not according to the claimed invention)

FIG. 1 is a top view of a composition in a non-interlocked configuration, according to certain embodiments.
FIG. 2 is a perspective view of a composition in a partially interlocked configuration, according to certain embodiments.
FIG. 3 is a perspective view of a composition in a fully interlocked configuration, according to certain embodiments.
FIG. 4 is a perspective view of two compositions forming a composition chain in an interlocked configuration, according to certain embodiments.
FIG. 5 is a perspective view of a composition in an interlocked configuration relative to surrounding tissue, according to certain embodiments.
FIG. 6 is a top view of a composition in a non-interlinked configuration, according to certain embodiments.
FIG. 7 is a top view of a composition in an interlocked configuration relative to surrounding tissue, according to certain embodiments.
FIG. 8 is a perspective view of a composition in a non-interlocked configuration, according to certain embodiments.
FIG. 9 is a top view of a composition in a non-interlocked configuration, according to certain embodiments.
FIG. 10 is a perspective view of a composition in an interlocked configuration, according to certain embodiments.
FIG. 11 is a perspective view of a composition in an interlocked configuration, according to certain embodiments.
FIG. 12 is a perspective view of two compositions forming a composition chain in an interlocked configuration, according to certain embodiments.
FIG. 13 is a perspective view of a composition in an interlocked configuration relative to surrounding tissue, according to certain embodiments.
FIG. 14 is a perspective view of compositions in an interlocked configuration, according to certain embodiments.
FIG. 15 is a perspective view of a composition in a non-interlocked configuration, according to certain embodiments.
FIG. 16 is a perspective view of compositions in an interlocked configuration relative to surrounding tissue, according to certain embodiments.
FIG. 17 is a perspective, cross-sectional view of a composition in an interlocked configuration relative to surrounding tissue and an anchoring material, according to certain embodiments.
FIG. 18 is a side, cross-sectional view of a composition in an interlocked configuration relative to surrounding tissue and an anchoring material, according to certain embodiments.
FIG. 19 is a perspective, cross-sectional view of a composition in an interlocked configuration relative to surrounding tissue and an anchoring material, according to certain embodiments.
FIG. 20 is a side, cross-sectional view of a composition in an interlocked configuration relative to surrounding tissue and an anchoring material, according to certain embodiments.
FIG. 21 is a perspective, cross-sectional view of a composition in an interlocked configuration relative to surrounding tissue and an anchoring material, according to certain embodiments.
FIG. 22 is a side, cross-sectional view of a composition in an interlocked configuration relative to surrounding tissue and an anchoring material, according to certain embodiments.
FIG. 23 is a side view of a composition in an interlocked configuration and surrounding an implant, according to certain embodiments.
FIG. 24 is a top view of a first portion of a composition, according to certain embodiments.
FIG. 25 is a top view of a second portion of a composition, according to certain embodiments.
FIG. 26 is a perspective view of a first portion and a second portion of a composition in an interlocked configuration and surrounding an implant, according to certain embodiments.
FIG. 27 is a perspective view of a first portion of a composition in an interlocked configuration relative to surrounding tissue and surrounding an implant, according to certain embodiments.

### DESCRIPTION OF CERTAIN EXEMPLARY EMBODIMENTS

Reference will now be made in detail to certain exemplary embodiments according to the present disclosure, certain examples of which are illustrated in the accompanying drawings. Wherever possible, the same reference numbers will be used throughout the drawings to refer to the same or like parts.

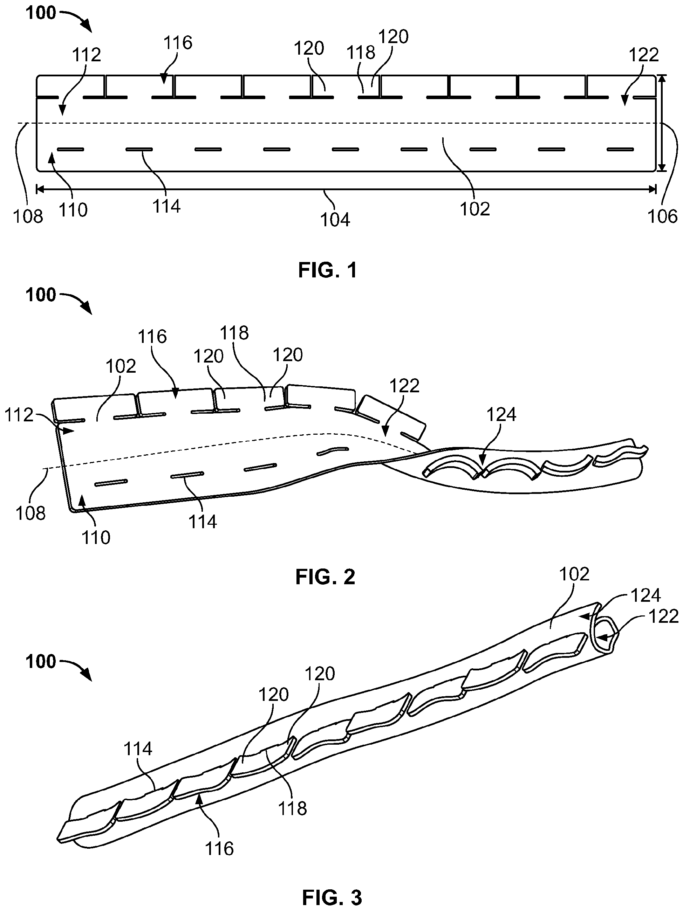

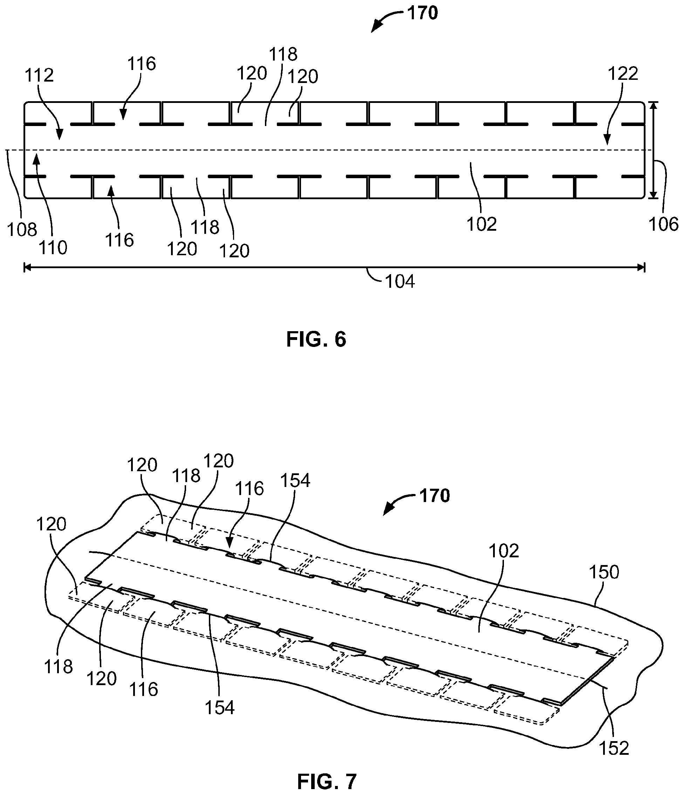

The herein after described embodiments relating to figures 1-5 are according to the claimed invention. The herein after described embodiments relating to figures 6-27 are reference embodiments, which do not fall within the scope of the claimed invention.

In this application, the use of the singular includes the plural unless specifically stated otherwise. In this application, the use of "or" means "and/or" unless stated otherwise. Furthermore, the use of the term "including", as well as other forms, such as "includes" and "included", is not limiting. Any range described herein will be understood to include the endpoints and all values between the endpoints.

The section headings used herein are for organizational purposes only and are not to be construed as limiting the subject matter described.

Various human and animal tissues can be used to produce products or compositions for treating patients. For example, various tissue products for regeneration, repair, augmentation, reinforcement, and/or treatment of human tissues that have been damaged or lost due to various diseases and/or structural damage (e.g., from trauma, surgery, atrophy, and/or long-term wear and degeneration) have been produced. Such products can include, for example, acellular tissue matrices, tissue allografts or xenografts, and/or reconstituted tissues (i.e., at least partially decellularized tissues that have been seeded with cells to produce viable materials).

In certain embodiments, these products or compositions can be completely or partially decellularized to yield acellular tissue matrices or extracellular tissue materials to be used for patients. For example, various tissues, such as skin, intestine, bone, cartilage, nerve tissue (e.g., nerve fibers or dura), tendons, ligaments, or other tissues can be completely or partially decellularized to produce tissue products useful for patients. In some cases, these decellularized products can be used without addition of exogenous cellular materials (e.g., stem cells). In certain cases, these decellularized products can be seeded with cells from autologous sources or other sources to facilitate treatment. Suitable processes for producing acellular tissue matrices are described below.

Tissue products can be selected to provide a variety of different biological and mechanical properties. For example, an acellular tissue matrix or other tissue product can be selected to allow tissue ingrowth and remodeling to assist in regeneration of tissue normally found at the site where the matrix is implanted. For example, an acellular tissue matrix, when implanted on or into fascia, may be selected to allow regeneration of the fascia without excessive fibrosis or scar formation. In certain embodiments, the tissue product can be formed from ALLODERM® or STRATTICE™, which are human and porcine acellular dermal matrices, respectively. Alternatively, other suitable acellular tissue matrices can be used, as described further below. The methods for shaping tissues having an extracellular matrix can be used to process any collagenous tissue type, and for any tissue matrix product. For example, a number of biological scaffold materials as described by Badylak et al., or any other similar materials, can be used to produce tissues with a stable three-dimensional shape. Badylak et al., "Extracellular Matrix as a Biological Scaffold Material: Structure and Function," Acta Biomaterialia (2008), doi:10.1016/j.actbio.2008.09.013. According to the present invention, the compositions discussed herein are formed from or include a tissue product.

The term "acellular tissue matrix," as used herein, refers generally to any tissue matrix that is substantially free of cells and/or cellular components. Skin, parts of skin (e.g., dermis), and other tissues such as blood vessels, heart valves, fascia, cartilage, bone, and nerve connective tissue may be used to create acellular matrices within the scope of the present disclosure. Acellular tissue matrices can be tested or evaluated to determine if they are substantially free of cell and/or cellular components in a number of ways. For example, processed tissues can be inspected with light microscopy to determine if cells (live or dead) and/or cellular components remain. In addition, certain assays can be used to identify the presence of cells or cellular components. For example, DNA or other nucleic acid assays can be used to quantify remaining nuclear materials within the tissue matrices. Generally, the absence of remaining DNA or other nucleic acids will be indicative of complete decellularization (i.e., removal of cells and/or cellular components). Finally, other assays that identify cell-specific components (e.g., surface antigens) can be used to determine if the tissue matrices are acellular.

In general, the steps involved in the production of an acellular tissue matrix include harvesting the tissue from a donor (e.g., a human cadaver or animal source) and cell removal under conditions that preserve biological and structural function. In certain embodiments, the process includes chemical treatment to stabilize the tissue and avoid biochemical and structural degradation together with or before cell removal. In various embodiments, the stabilizing solution arrests and prevents osmotic, hypoxic, autolytic, and proteolytic degradation, protects against microbial contamination, and reduces mechanical damage that can occur with tissues that contain, for example, smooth muscle components (e.g., blood vessels). The stabilizing solution may contain an appropriate buffer, one or more antioxidants, one or more oncotic agents, one or more antibiotics, one or more protease inhibitors, and/or one or more smooth muscle relaxants.

The tissue is then placed in a decellularization solution to remove viable cells (e.g., epithelial cells, endothelial cells, smooth muscle cells, and fibroblasts) from the structural matrix without damaging the biological and structural integrity of the collagen matrix. The decellularization solution may contain an appropriate buffer, salt, an antibiotic, one or more detergents (e.g., TRITON X-100™, sodium deoxycholate, polyoxyethylene (20) sorbitan mono-oleate), one or more agents to prevent cross-linking, one or more protease inhibitors, and/or one or more enzymes. In some embodiments, the decellularization solution comprises 1% TRITON X-100™ in RPMI media with Gentamicin and 25 mM EDTA (ethylenediaminetetraacetic acid). In some embodiments, the tissue is incubated in the decellularization solution overnight at 37 ºC with gentle shaking at 90 rpm. In certain embodiments, additional detergents may be used to remove fat from the tissue sample. For example, in some embodiments, 2% sodium deoxycholate is added to the decellularization solution.

After the decellularization process, the tissue sample is washed thoroughly with saline. In some exemplary embodiments, e.g., when xenogenic material is used, the decellularized tissue is then treated overnight at room temperature with a deoxyribonuclease (DNase) solution. In some embodiments, the tissue sample is treated with a DNase solution prepared in DNase buffer (20 mM HEPES (4-(2-hydroxyethyl)-1-piperazineethanesulfonic acid), 20 mM CaCl₂ and 20 mM MgCl₂). Optionally, an antibiotic solution (e.g., Gentamicin) may be added to the DNase solution. Any suitable buffer can be used as long as the buffer provides suitable DNase activity.

While an acellular tissue matrix may be made from one or more individuals of the same species as the recipient of the acellular tissue matrix graft, this is not necessarily the case. Thus, for example, an acellular tissue matrix may be made from porcine tissue and implanted in a human patient. Species that can serve as recipients of acellular tissue matrix and donors of tissues or organs for the production of the acellular tissue matrix include, without limitation, mammals, such as humans, nonhuman primates (e.g., monkeys, baboons, or chimpanzees), pigs, cows, horses, goats, sheep, dogs, cats, rabbits, guinea pigs, gerbils, hamsters, rats, or mice.

Elimination of the α-gal epitopes from the collagen-containing material may diminish the immune response against the collagen-containing material. The α-gal epitope is expressed in non-primate mammals and in New World monkeys (monkeys of South America) as well as on macromolecules such as proteoglycans of the extracellular components. U. Galili et al., J. Biol. Chem. 263: 17755 (1988). This epitope is absent in Old World primates (monkeys of Asia and Africa and apes) and humans, however. *Id.* Anti-gal antibodies are produced in humans and primates as a result of an immune response to α-gal epitope carbohydrate structures on gastrointestinal bacteria. U. Galili et al., Infect. Immun. 56: 1730 (1988); R. M. Hamadeh et al., J. Clin. Invest. 89: 1223 (1992).

Since non-primate mammals (e.g., pigs) produce α-gal epitopes, xenotransplantation of collagen-containing material from these mammals into primates often results in rejection because of primate anti-Gal binding to these epitopes on the collagen-containing material. The binding results in the destruction of the collagen-containing material by complement fixation and by antibody dependent cell cytotoxicity. U. Galili et al., Immunology Today 14: 480 (1993); M. Sandrin et al., Proc. Natl. Acad. Sci. USA 90: 11391 (1993); H. Good et al., Transplant. Proc. 24: 559 (1992); B. H. Collins et al., J. Immunol. 154: 5500 (1995). Furthermore, xenotransplantation results in major activation of the immune system to produce increased amounts of high affinity anti-gal antibodies. Accordingly, in some embodiments, when animals that produce α-gal epitopes are used as the tissue source, the substantial elimination of α-gal epitopes from cells and from extracellular components of the collagen-containing material, and the prevention of re-expression of cellular α-gal epitopes can diminish the immune response against the collagen-containing material associated with anti-gal antibody binding to α-gal epitopes.

To remove α-gal epitopes, after washing the tissue thoroughly with saline to remove the DNase solution, the tissue sample may be subjected to one or more enzymatic treatments to remove certain immunogenic antigens, if present in the sample. In some embodiments, the tissue sample may be treated with an α-galactosidase enzyme to eliminate α-gal epitopes if present in the tissue. Any suitable enzyme concentration and buffer can be used as long as sufficient removal of antigens is achieved.

Alternatively, rather than treating the tissue with enzymes, animals that have been genetically modified to lack one or more antigenic epitopes may be selected as the tissue source. For example, animals (e.g., pigs) that have been genetically engineered to lack the terminal α-galactose moiety can be selected as the tissue source. For descriptions of appropriate animals see co-pending U.S. Application Serial No. 10/896,594 and U.S. Patent No. 6,166,288. In addition, certain exemplary methods of processing tissues to produce acellular matrices with or without reduced amounts of or lacking alpha-1,3-galactose moieties, are described in Xu, Hui. et al., "A Porcine-Derived Acellular Dermal Scaffold that Supports Soft Tissue Regeneration: Removal of Terminal Galactose-α-(1,3)-Galactose and Retention of Matrix Structure," Tissue Engineering, Vol. 15, 1-13 (2009).

After the acellular tissue matrix is formed, histocompatible, viable cells may optionally be seeded in the acellular tissue matrix to produce a graft that may be further remodeled by the host. In some embodiments, histocompatible viable cells may be added to the matrices by standard in vitro cell co-culturing techniques prior to transplantation, or by in vivo repopulation following transplantation. In vivo repopulation can be by the recipient's own cells migrating into the acellular tissue matrix or by infusing or injecting cells obtained from the recipient or histocompatible cells from another donor into the acellular tissue matrix in situ. Various cell types can be used, including embryonic stem cells, adult stem cells (e.g. mesenchymal stem cells), and/or neuronal cells. In various embodiments, the cells can be directly applied to the inner portion of the acellular tissue matrix just before or after implantation. In certain embodiments, the cells can be placed within the acellular tissue matrix to be implanted, and cultured prior to implantation.

With reference to FIGS. 1-5, one embodiment of an exemplary composition 100 in a non-interlocked, a partially interlocked, a fully interlocked, a fully interlocked chain, and a fully interlocked with tissue configuration, respectively, is shown. The composition 100 includes a composition body 102. In certain embodiments, the composition body 102 can be shaped or formed into a substantially flat configuration. Although illustrated as a rectangular composition body 102, it should be understood that the composition body 102 can be formed into a variety of shapes. In addition, it should be understood that the composition body 102 can initially be provided as defining one shape and can further be trimmed or formed by a user to vary the shape of the composition body 102.

The composition body 102 generally defines an elongated length 104 and a width 106. The composition body 102 can define a variety of lengths 104, widths 106, or both. In particular, those of ordinary skill in the art should understand that the length 104, the width 106, or both, can be varied to accommodate wrapping the composition 100 around differently dimensioned bones or tissue. For example, a bone having a wide diameter and a long length may require a composition body 102 defining a longer length 104 and a larger width 106 as compared to a bone having a small diameter and a short length.

A centerline 108 extending the length 104 of the composition body 102 can separate the composition body into a first half 110 and a second half 112. In certain embodiments, the first half 110 and the second half 112 of the composition body 102 can be dimensioned substantially equal relative to each other. In certain embodiments, the first half 110 and the second half 112 of the composition body 102 can be dimensioned unequally relative to each other. For example, an area defined by the first half 110 can be substantially unequal relative to an area defined by the second half 112. In certain embodiments, rather than extending parallel to the sides of the composition body 102, the centerline 108 can extend at an angle relative to the sides of the composition body 102.

The composition body 102 includes one or more first components 114 formed therein. In particular, the first components 114 can be formed in the first half 110. The first components 114 can include one or more holes or slits, e.g., a series of holes or slits, passing through the composition body 102. In certain embodiments, the first components 114 can be formed to extend parallel relative to a side of the composition body 102 and/or the centerline 108. In certain embodiments, a frequency of first component 114 formation in the composition body 102, e.g., the spacing between each first component 114, can vary.

As will be described in greater detail below, the first components 114 can be configured and dimensioned to interlock with respective second components 116 formed in the composition body 102. In particular, one or more second components 116 can be formed in the second half 112 such that the second components 116 are positioned in an opposing and aligned relation to the respective first components 114 across the centerline 108. Thus, each second component 116 can correspond to and potentially be interlocked relative to an opposing first component 114.

In certain embodiments, each of the second components 116 includes a tab 118, e.g., a T-shaped tab, extending substantially perpendicularly relative to the centerline 108 and/or the length 104 of the elongated body 102 and two opposing extensions 120 extending from the tab 118. For example, the tab 118 can extend from the composition body 102 in a direction perpendicular to and away from the centerline 108. A proximal end of the tab 118 can be connected to the composition body 102 and located closer relative to the centerline 108 as compared to a distal end of the tab 118 which connects to the two opposing extensions 120. Thus, the tab 118 can movably connect the two opposing extensions 120 to the composition body 102.

The two opposing extensions 120 can extend from the tab 118 in opposing directions substantially parallel to the centerline 108. In certain embodiments, the tab 118 can be dimensioned substantially similar or slightly less in length than the second component 114. Although illustrated as square or rectangular extensions 120, in certain embodiments, a variety of configurations can be utilized. In certain embodiments, the second components 116 can be formed in the composition body 102 by forming T-shaped cuts along the length 104 of the composition body 102 as illustrated in FIG. 1.

The first and the second components 114, 116 can be formed to be complementary relative to each other. In addition, the first and the second components 114, 116 can be configured to interlock relative to each other. For example, the composition 100 can be formed of materials which allow bending or flexibility of the composition 100 such that the composition 100 can be wrapped around a tissue or bone and the first and second components 114, 116 can be interlocked relative to each other. The composition 100 can thereby be positioned around tissue or bone and the first and second components 114, 116 can be interlocked relative to each other to maintain the composition 100 in the desired position without sutures.

According to certain embodiments, methods of interlocking the first and second components 114, 116 include bending or folding the two opposing extensions 120 over each other adjacent to the tab 118 into a non-extending configuration. For example, a first opposing extension 120 can be folded parallel to the centerline 108 in the direction of the tab 118 and a second opposing extension 120 can be folded over the first opposing extension 120 such that in the non-extended configuration, the two opposing extensions 120 define a length substantially similar to the length of the tab 118. While in the non-extended configuration, the two opposing extensions 120 of the second component 116 can be passed through a corresponding hole or slit of the first component 114, where passage through the first component 114 defines passage in a first direction. In particular, passage in the first direction can be defined by passage of the second component 116 from an inner surface 122 of the composition body 102, through the first component 114 and out at an outer surface 124 opposing the inner surface 122. It should be understood that as referred to herein, the hole or slit of the first component 114 can be a cut along a line or a region in which material has been removed to provide a passage therethrough.

Once the two opposing extensions 120 have been fully passed through the first component 114, the two opposing extensions 120 can be unbent or unfolded into an extended configuration. Thus, the tab 118 can be positioned within the first component 114 (or passing through the first component 114 and extending out on either side of the composition body 102) and the two opposing extensions 120 can be positioned against the outer surface 124 of the composition body 102 to maintain the first and second components 114, 116 interlocked. In particular, the configuration and position of the two opposing extensions 120 prevents undesired passage or retraction of the two opposing extensions 120 in a second direction, e.g., through the first component 114 from the outer surface 124 to the inner surface 122. The composition 100 can thereby be interlocked around a tissue, tendon, nerve and/or bone and, if desired, can be removed from the tissue, tendon, nerve and/or bone by refolding the two opposing extensions 120 and passing the second component 116 in the second direction through the first component 114.

Although illustrated as including one row of first components 114, in certain embodiments, the composition 100 can include multiple rows of first components 114 variably positioned relative to the centerline 108 such that the first and second components 114, 116 can be interlocked at different positions along the length of the composition 100 to conform to the tissue, nerve, tendon or bone around which the composition 100 is positioned. By providing multiple rows of first components 114, the tightness with which the composition 100 is wrapped against tissue or bone can be adjusted.

In certain embodiments, in the interlocked configuration, the composition 100 can wrap around the tissue, nerve, tendon or bone approximately 360 degrees with substantially no overlapping of the composition body 102. In certain embodiments, in the interlocked configuration, the composition 100 can wrap around the tissue, nerve, tendon or bone more than 360 degrees such that the composition body 102 overlaps prior to the first and second components 114, 116 interlocking. For example, the composition body 102 can overlap to form a fluid-holding lumen.

FIG. 2 shows the composition 100 in a partially interlocked configuration and FIG. 3 shows the composition 100 in a fully interlocked configuration. In particular, the first and second components 114, 116 can be interlocked one-by-one by a user until the composition 100 is in a fully interlocked configuration. In the fully interlocked configuration, the composition 100 can define a substantially tubular form. Therefore, the composition 100 can be positioned adjacent to tissue or bone and the first and second components 114, 116 can be interlocked one-by-one until the composition 100 is wrapped around the desired portion of the tissue or bone. It should be understood that due to the flexible characteristics of the material of the composition 100, e.g., a tissue matrix, a synthetic material, combinations thereof, and the like, the composition 100 can be wrapped around non-linear tissue or bone by bending and/or twisting the composition 100 as needed to conform the composition 100 to the configuration of the tissue or bone.

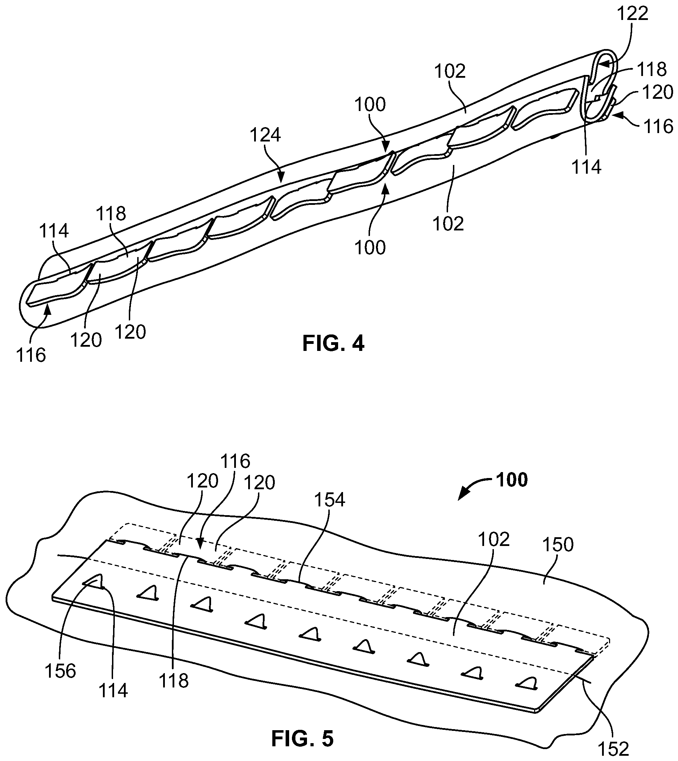

FIG. 4 shows two compositions 100 in a fully interlocked and chain configuration. In particular, rather than interlocking the first and second components 114, 116 of one composition 100 relative to each other, in certain embodiments, two or more compositions 100 can be interlocked relative to each other to increase the diameter of the compositions 100 in the interlocked configuration. For example, as shown in FIG. 4, the first components 114 of the first composition 100 can be fully interlocked with the second components 116 of the second composition 100, and the first components 114 of the second composition 100 can be fully interlocked with the second components 116 of the first composition 100. By increasing the diameter of the interlocked compositions 100, the compositions 100 can be wrapped around tissue or bone with a larger cross-sectional area or diameter.

FIG. 5 shows a composition 100 in a fully interlocked with tissue configuration. In particular, in certain embodiments, rather than interlocking the first and second components 114, 116 relative to each other, the first and second components 114, 116 can be interlocked relative to surrounding tissue 150. In certain embodiments, the composition 100 can be interlocked to the surrounding tissue 150 to close a surgical incision 152 in the tissue 150, maintain the surgical incision 152 closed, or both.

For example, after surgery is performed, closure of the surgical incision 152 is generally desired. Incisions 154 can be formed at a position offset from the surgical incision 152. It should be understood that as referred to herein, the incisions 154 can be a cut along a line or a region in which material has been removed to provide a passage therethrough. In particular, each of the incisions 154 can be configured and dimensioned to receive therein a second component 116 of the composition 100. In certain embodiments, the length of the incisions 154 can be dimensioned between the distance from one opposing extension 120 to the other opposing extension 120, and the width of the tab 118. The opposing extensions 120 can be folded onto each other and the second component 116 can be inserted into the incision 154 and under the tissue 150. The opposing extensions 120 can further be expanded under the tissue 150 to prevent the second components 116 from exiting the incisions 154.

The composition 100 can be positioned over the surgical incision 152 and the tissue 150 can be pulled as desired to substantially close the surgical incision 152. The first components 114 can further be interlocked relative to the surrounding tissue 150 by pulling portions of tissue 156 through the first components 114. The first components 114 can thereby be anchored to the surrounding tissue 152 and can maintain the surgical incision 152 in a closed position. Although illustrated as forming an incision 154 for each of the second components 116 and anchoring each of the first components 114 relative to the tissue 150, in certain embodiments, only a portion of the first and second components 114, 116 can be anchored to the tissue 150. However, it should be understood that the appropriate number of first and second components 114, 116 are anchored to the tissue 150 to ensure that sufficient tension is formed in the tissue 150 to maintain the surgical incision 152 in a closed configuration.

FIGS. 6 and 7 show a top view and a perspective view of an alternative embodiment of an exemplary composition 170. In particular, FIG. 6 shows a top view of the composition 170 in a non-interlocked configuration and FIG. 7 shows the composition 170 in a fully interlocked with tissue configuration. It should be understood that the composition 170 can be substantially similar in structure and function to the composition 100, except for the distinctions noted herein. Therefore, like structures are marked with like reference numbers.

In certain embodiments, rather than including a set of first components 114 and a set of complementary second components 116, the composition 170 can include two opposing sets of second components 116. For example, the sets of second components 116 can be symmetrically positioned on opposing sides of the centerline 108. The second components 116 can thereby extend outwardly away from the centerline 108 on each side of the composition body 102. In certain embodiments, the two sets of second components 116 can be positioned in an offset manner relative to each other. For example, a first set of second components 116 positioned on the first half 110 can be offset in a direction parallel to the centerline 108 relative to the second set of second components 116 positioned on the second half 112 of the composition 100.

As shown in FIG. 7, similar to the application of the composition 100 of FIG. 5, the composition 170 can be used to close and/or maintain a surgical incision 152 in tissue 150. For example, two sets of incisions 154 can be formed on each side of the surgical incision 152. The positions of the incisions 154 can correspond to the position or spacing of the second components 116 on each side of the composition 170. As discussed above, the dimensions of the incisions 154 can be such that the tab 118 and the two opposing extensions 120 can be at least partially inserted into the incision 154 and the opposing extensions 120 can be expanded to retain the second component 116 under the tissue 150. As shown in FIG. 7, by securing each of the second components 116 to the tissue 150 through the incisions 154 surrounding the surgical incision 152, the surgical incision 152 can be closed and/or maintained in a closed position to assist in patient recovery.

Although the composition 100 does not provide a liquid-tight closure around the tissue or bone, the composition 100 provides a protective sleeve that can be inserted in the desired location for an application that does not allow for a slip-over type of product. In addition, the composition 100 can be positioned onto tissue or bone without the implementation of sutures, thereby reducing the time and potential complications with application of the composition 100.

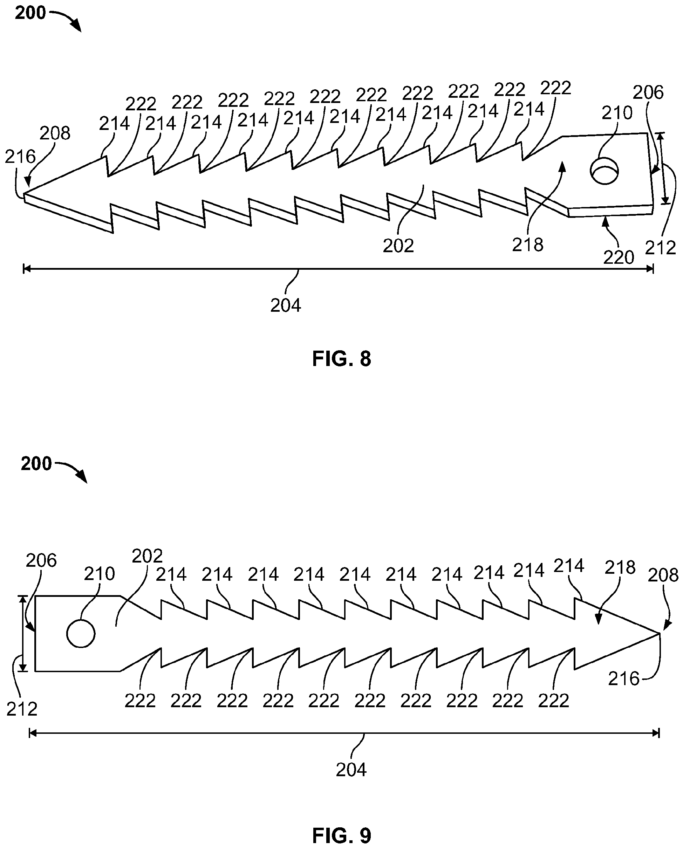

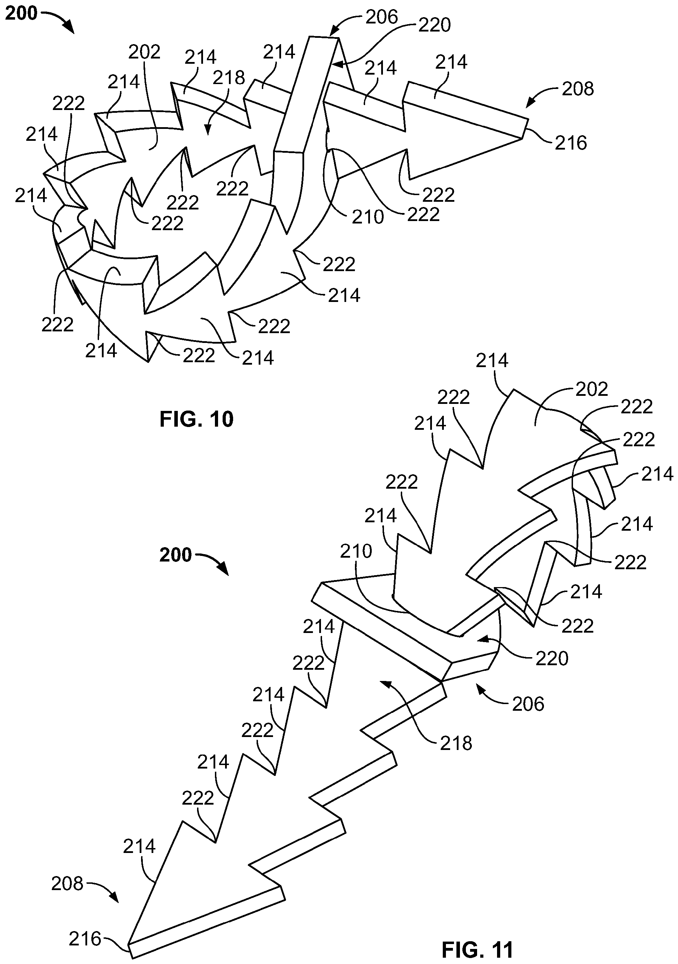

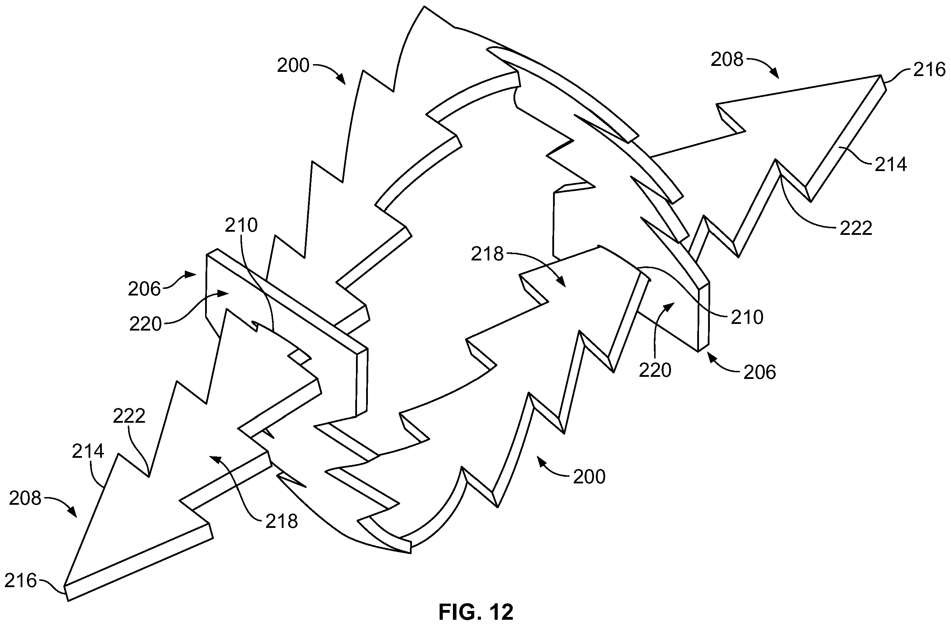

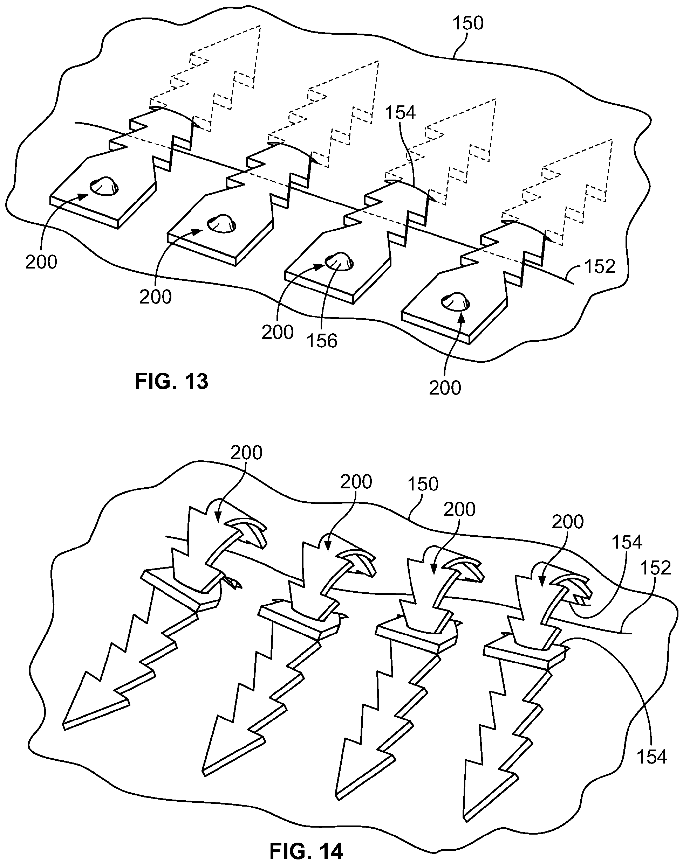

FIGS. 8-14 show an alternative embodiment of an exemplary composition 200. In particular, FIGS. 8 and 9 show the composition 200 in a non-interlocked configuration, FIGS. 10 and 11 show the composition 200 in an interlocked configuration, FIG. 12 shows the composition 200 in an interlocked chain configuration, and FIGS. 13 and 14 show compositions 200 in an interlocked configuration relative to surrounding tissue.

The composition 200 includes a composition body 202. In certain embodiments, the composition body 202 can be shaped or formed into a substantially flat configuration. In certain embodiments, the composition body 202 can be shaped or formed in a substantially cylindrical or circular configuration. It should be understood that although the composition body 202 can initially define an elongated length 204, the composition body 202 can be trimmed by a user to vary the length 204 of the composition body 202. In certain embodiments, compositions 200 of a variety of lengths 204 can be provided.

The composition body 202 can define a proximal end 206 and a distal end 208 located on opposite ends of the composition body 202. The proximal end 206 includes a first component 210, e.g., an opening, a restricted space, and the like, passing therethrough. The first component 210 can be spaced from the edge of the proximal end 206 and can be centrally positioned along the width 212 of the composition body 202. Although illustrated as being formed in a portion having three surrounding perpendicular sides and one side including two angled portions, it should be understood that the first component 210 can be formed in a portion having substantially perpendicular sides all around.

As will be described in greater detail below, the first component 210 can be configured and dimensioned to interlock with respect to second components 214 formed in the composition body 202. In particular, a plurality of second components 214, e.g., flared sections, tabs, and the like, can be formed along the length 204 of the composition body 202. The second components 214 can extend from a point adjacent to the first component 210 to the distal end 208 of the composition body 202. Each second component 214 can include two flared sections or edges extending on opposing sides of the composition body 202. For example, as illustrated in FIG. 8, the second component 214 can define substantially triangular edges. In certain embodiments, the second component 214 positioned at the distal end 208 of the composition body 202 can include a pointed or arrow-like tip 216 to assist in interlocking the second component 214 at the distal end 208 to the first component 210.

The first and second components 210, 214 can be formed to be complementary relative to each other. In particular, the first and second components 210, 214 can be configured to interlock relative to each other. For example, the composition 200 can be formed of materials which allow bending or flexibility of the composition body 202 such that the distal end 208, e.g., the tip 216, can be aligned with the first component 210 and one or more of the second components 214 can be slipped or passed through the first component 210 in a first direction defined by passage from the inner surface 218, through the first component 210 and to the outer surface 220. The flared tips of the second component 214 can be positioned against the outer surface 220 of the composition body 202 and prevent pull-back, retraction or passage of the second component 214 out of the first component 210 in a second direction, e.g., a direction defined by passage from the outer surface 220, through the first component 210 and to the inner surface 218.

According to certain embodiments, methods of interlocking the first and second components 210, 214 include passing one or more of the second components 214 through the first component 210 such that the flared tips extending from the second components 214 prevent the second component 214 from being removed from the first component 210. The first and second components 210, 214 can thereby be interlocked relative to each other.

Examples of the composition 200 in an interlocked configuration are shown in FIGS. 10 and 11. In particular, the composition 200 of FIG. 10 is shown with the first component 210 interlocked between the second and third second components 214 (from the distal end 208). The composition 200 of FIG. 11 is shown with the first component 210 interlocked between the fourth and fifth second components 214 (from the distal end 208). It should be understood that the tightness with which the composition 200 can be secured around bone or tissue depends on how far the distal end 208 of the composition 200 is passed through the first component 210. Thus, the composition 200 of FIG. 10 can provide a tighter grip around bone or tissue, or can be positioned around bone or tissue defining a smaller diameter or cross-section, as compared to the composition 200 of FIG. 11.

In certain embodiments, as shown in FIG. 12, two or more compositions 200 can be interlocked relative to each other in a chain configuration to increase the cross-section around which the compositions 200 can be secured. For example, rather than interlocking the first and second components 210, 214 of one composition relative to each other, the first component 210 of the first composition 200 can be interlocked relative to the second component 214 of the second composition 200, and the first component 210 of the second composition 200 can be interlocked relative to the second component 214 of the first composition 200. Although two compositions 200 are shown in FIG. 12, it should be understood that depending on the application, any number of compositions 200 can be used. In addition, rather than forming a continuous chain, multiple compositions 200 can be interlocked at varying angles to produce compositions having any suitable shape or size (e.g., in a T-shape, X-shape, spider-like shape, or in the form of a polyhedron or three-dimensional structure).

In certain embodiments, the second components 214 can be passed through the first component 210 more than one time, e.g., double interlocking, double-back interlocking, and the like. For example, the second component 214 can be passed through the first component 210 a first time, the composition body 202 can be wrapped around the tissue, tendon, nerve or bone a second time, and the second component 214 can be passed through the first component 210 a second time to provide for a stronger closure or interlocking. As a further example, the second component 214 can be passed through the first component 210 a first time and can be bent back in the direction of the first component 210 to pass the second component 214 through the first component 210 a second time, thereby providing for a stronger closure or interlocking.

In certain embodiments, the composition 200 can be used to interlock one or more tissues, tendons, nerves, or bones relative to each other. For example, the composition body 202 can be at least partially wrapped around one or more tissues, tendons, nerves, or bones and the first and second components 210, 214 can be interlocked to retain or bind the tissues, bones, nerves, or tendons in the desired position relative to each other. By providing a plurality of second components 214, the tightness with which the tissues, tendons, nerves, or bones are bound can be adjusted.

In certain embodiments, the composition 200 can be used to close incisions, e.g., abdominal tissue incisions, by passing and partially wrapping the composition body 202 through formed openings in the tissue and interlocking the first and second components 210, 214 relative to each other to prevent the two opposing tissues from being separated. By providing a plurality of second components 214, the tightness or gap between the tissues can be regulated. It should be understood that once a user has interlocked the first and second components 210, 214, the portion of the distal end 208 protruding from the first component 210 can be trimmed to compactly position the composition 200. Tissues, tendons, nerves, bones, or combinations thereof, can thereby be bound without the use of sutures.

For example, FIGS. 13 and 14 show a plurality of compositions 200 in an interlocked configuration with surrounding tissue 150. In particular, rather than interlocking the first and second components 210, 214 relative to each other, FIG. 13 shows the first and second components 210, 214 interlocked or anchored relative to the surrounding tissue 150 to close and/or maintain a surgical incision 152 closed. Depending on the size or length of the surgical incision 152, one or more incisions 154 can be formed along one side of the surgical incision 152 substantially parallel to the length of the surgical incision 152. The incisions 154 can be configured and dimensioned to receive therethrough one or more second components 214 of the composition 200 such that the barbs of the second components 214 prevent the distal end 208 of the composition 200 from retracting out of the incision 154. In particular, one or more second components 214 can be passed through the incision 154 and under the tissue 150 to anchor the distal end 208 of the composition 200 to the tissue 150. The proximal end 206 can be anchored to the tissue 150 by pulling a portion of tissue 156 through the first component 210. The compositions 200 can thereby stretch across the surgical incision 152 to maintain the surgical incision 152 closed.

FIG. 14 shows a plurality of compositions 200 passed through incisions 154 formed around a surgical incision 152 and interlocked relative to each other. In particular, two sets of opposing incisions 154 can be formed substantially symmetrically on either side of the surgical incision 152. The incisions 154 can create a passage under the tissue 150 between the two sets of incisions 154. The distal end 208 of each composition 200 can be passed through a first incision 154 on one side of the surgical incision 150, under the tissue 150 and the surgical incision 152, and through a second incision 154 on the opposing side of the surgical incision 152. The second component 214 can further be interlocked relative to the first component 210. Depending on the desired amount of closure of the surgical incision 152, the distal end 208 of the composition 200 can be pulled further through the first component 210 to tighten the interlocked configuration of the composition 200. Although shown as using four compositions 200, it should be understood that the number of compositions 200 can be determined based on the size or length of the surgical incision 152. In certain embodiments, after the first and second components 210, 214 have been interlocked, a portion of the distal end 208 can be trimmed to reduce the amount of material extending around the surgical incision 152.

The composition 200 can retain tightness in the interlocked configuration by preventing the second component 214 from retreating through the first component 210, while having sufficient strength to prevent breakage of the composition body 202 through material failure at the narrow sections of the composition 200. Table 1 below provides results of experimental testing performed to evaluate the force required to pull the second component 214 back through the first component 210 once the second component 214 had been fully pulled through the first component 210.

**TABLE 1: Pull Force Experimental Results**

| **Pliable/Firm** | **Hole Size (mm)** | **Sample No.** | **Max Load (N)** | **Avg. Max Load (N)** | **St. Dev.** |
|---|---|---|---|---|---|
| Pliable | 2 | 1 | 6.11 | | |
| Pliable | 2 | 2 | 5.16 | 4.94 | 1.30 |
| Pliable | 2 | 3 | 3.54 | | |
| Pliable | 3 | 1 | 2.81 | | |
| Pliable | 3 | 2 | 0.93 | 2.59 | 1.57 |
| Pliable | 3 | 3 | 4.04 | | |
| Firm | 3 | 1 | 9.55 | | |
| Firm | 3 | 2 | 12.25 | 9.63 | 2.58 |
| Firm | 3 | 3 | 7.10 | | |

With respect to Table 1, "Pliable/Firm" represents the rigidity of the composition 200 tested, "Hole Size" represents the diameter of the first component 210, "Sample No." represents each one of the three samples of the composition 200 which was tested per group, "Max Load" represents the maximum load in Newtons at which the second component 214 can be pulled back through the first component 210, "Avg. Max Load" represents the average maximum load in Newtons for each group of three sample compositions 200 tested, and "St. Dev." represents the standard deviation for the average maximum load.

In certain embodiments, the configuration of the composition 200 can be altered to increase the load required to pull open the composition 200, e.g., pull the second component 214 out of the first component 210. For example, the width 212 of the extended flares of the second component 214, the diameter, dimension or configuration of the first component 210, the number of interlocking second components 214, and the like, can be varied to adjust the load for pulling the second component 214 out of the first component 210.

Additional experimentation was performed to evaluate the strength of narrow points 222 in the composition 200. In particular, the connecting points between the second components 214 and the connecting point between the second component and the proximal end 206 can define narrower points 222 than the remaining portions of the composition body 202. The data provided in Table 2 below shows that the material strength was greater at the narrow points 222 than the loads for releasing the second component 214 from the first component 210 as provided in Table 1.

**TABLE 2: Narrow Points Experimental Results**

| **Width (mm)** | **Sample No.** | **Max Load (N)** | **Avg. Max Load (N)** | **St. Dev.** |
|---|---|---|---|---|
| | 1 | 83.09 | | |
| 3 | 2 | 129.31 | 104.40 | 23.32 |
| | 3 | 100.79 | | |
| | 1 | 100.81 | | |
| 4 | 2 | 125.80 | 111.48 | 12.89 |
| | 3 | 107.84 | | |
| | 1 | 125.94 | | |
| 5 | 2 | 152.04 | 130.68 | 19.43 |
| | 3 | 114.06 | | |
| | 1 | 130.12 | | |
| 7 | 2 | 173.75 | 143.62 | 26.14 |
| | 3 | 126.99 | | |

With respect to Table 2, "Width" represents the width at the narrow point 222, "Sample No." represents each one of the three samples of the composition 200 which was tested per group, "Max Load" represents the maximum load in Newtons for material failure at the narrow point 222, "Avg. Max Load" represents the average maximum load in Newtons for each group of three sample compositions 200 tested, and "St. Dev." Represents the standard deviation for the average maximum load.

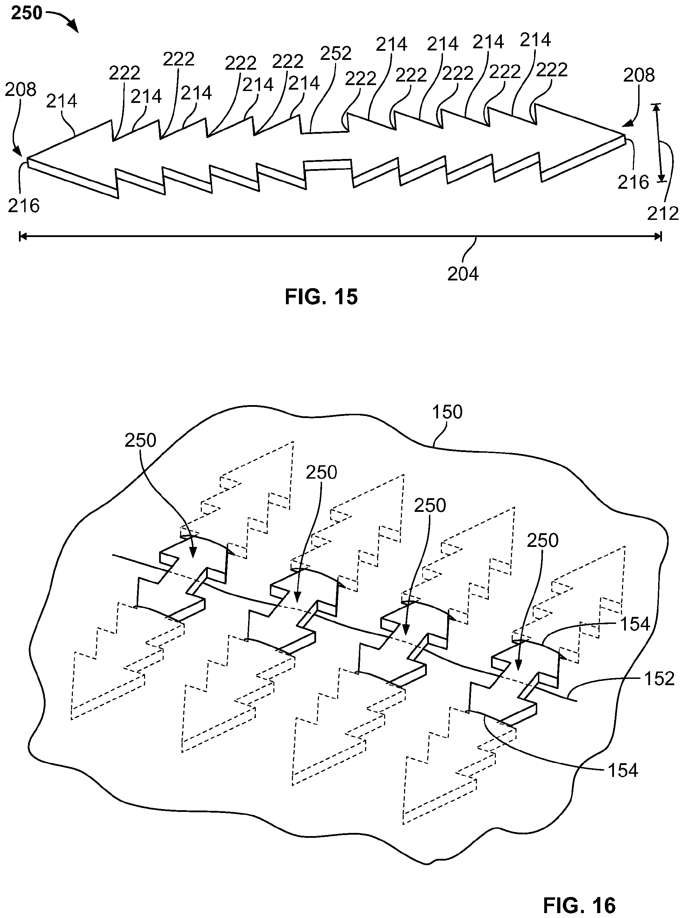

With reference to FIGS. 15 and 16, perspective views of an alternative embodiment of an exemplary composition 250 are provided. In particular, FIG. 15 shows a perspective view of the composition 250 in a non-interlocked configuration and FIG. 16 shows a perspective view of a plurality of compositions 250 in an interlocked configuration relative to surrounding tissue. It should be understood that the composition 250 can be substantially similar in structure and function to the composition 200, except for the distinctions noted herein. Therefore, like structures are marked with like reference numbers.

Rather than including a first component 210 and second components 214, the composition 250 can include two sets of second components 214. In particular, the distal ends 208 of the second components 214 can extend outwardly away from each other and can be connected by a central portion 252. In certain embodiments, the second components 214 can extend in a substantially aligned and parallel manner relative to each other. In certain embodiments, the second components 214 can extend in an unaligned and angled manner relative to each other. Each of the second components 214 includes an arrow-like tip 216 at the distal end 208 for interlocking to tissue 150.

For example, FIG. 16 shows a plurality of compositions 250 interlocked or anchored relative to tissue 150 surrounding a surgical incision 152. Depending on the number of compositions 250 used, a set of incisions 154 can be formed in the tissue 150 on either side of the surgical incision 152. The incisions 154 can be configured and dimensioned to receive therein at least one of the second components such that the distal end 208 of the composition 250 can be anchored to the tissue 150. As shown in FIG. 16, each distal end 208 of the composition 250, as well as three second components 216, have been inserted through the incisions 154 and under the tissue 150 to close the surgical incision 152 and anchor the composition 250 to the tissue 150. The surgical incision 152 can thereby be maintained closed post-surgery. It should be understood that one or both distal ends 208 of the composition 250 can be trimmed by a surgeon to reduce the number of second components 214 positioned beneath the tissue 150.

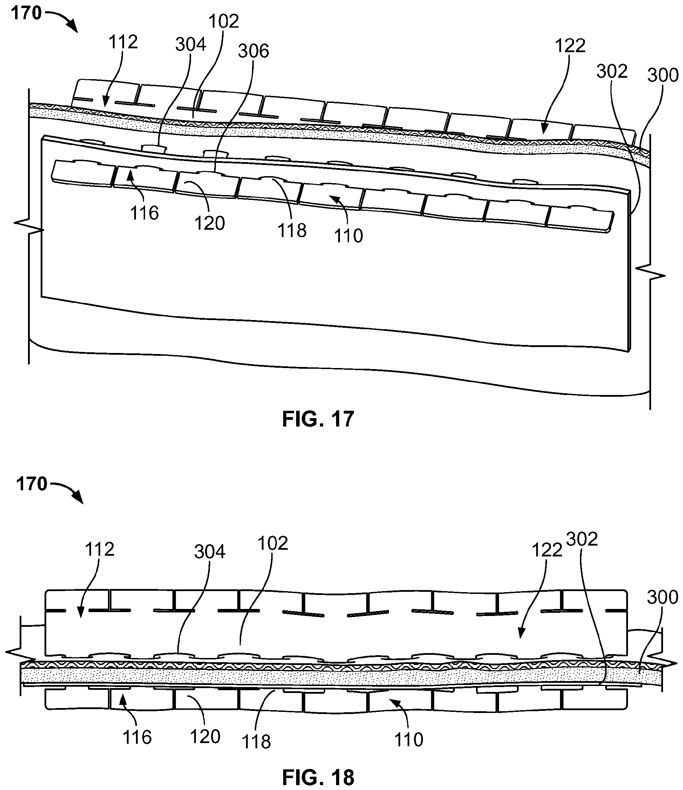

With reference to FIGS. 17 and 18, perspective and side cross-sectional views of an alternative embodiment of an exemplary composition 170 are provided. In particular, FIG. 17 shows a perspective, cross-sectional view of the composition 170 in an interlocked configuration with surrounding tissue 300, and FIG. 18 shows a side, cross-sectional view of the composition 170 in an interlocked configuration relative to the surrounding tissue 300. It should be understood that the composition 170 can be substantially similar in structure and function to the composition 170 discussed with respect to FIGS. 6 and 7. Although illustrated with composition 170, it should be understood that in certain embodiments, composition 100 can be used in a substantially similar manner.

In particular, rather than interlocking the composition 170 to itself or only to the surrounding tissue 300, in certain embodiments, the composition 170 can be interlocked relative to an anchoring material 302, e.g., a separate piece of material. In certain embodiments, the anchoring material 302 can be formed from a biocompatible material, such as tissue. The anchoring material 302 can act as a fixation material or a bolt to maintain the composition 170 secured to the surrounding tissue 300. Although illustrated as including an anchoring material 302 positioned adjacent to the surrounding tissue 300, it should be understood that in certain embodiments, additional materials can be positioned between, e.g., the anchoring material 302 and the surrounding tissue 300, the surrounding tissue 300 and the composition 170, the anchoring material 302 and the composition 170, combinations thereof, or the like.

As an example, the surrounding tissue 300 can include a plurality of openings 304 formed therein and passing therethrough. The openings 304 can be complementary to the tabs 118 of the composition 170. In particular, each of the openings 304 can be configured and dimensioned to at least partially receive therethrough an individual tab 118 as shown in FIGS. 17 and 18.

The anchoring material 302 can also include a plurality of openings 306 formed therein and passing therethrough. The anchoring material 302 can be positioned on an opposing side of the surrounding tissue 300 relative to the composition 170 and the openings 306 of the anchoring material 302 can be aligned with the openings 304 of the surrounding tissue 300.

The opposing extensions 120 of the tabs 118 can be bent or folded over each other into a non-extending configuration such that the tabs 118 can be passed through the openings 304, 306. Further, the individual tabs 118 can be at least partially passed through the respective openings 304, 306 until the tabs 118 extend through the surrounding tissue 300 and the anchoring material 302. The tabs 118 can thereby extend from one side (e.g., an exterior side) of the surrounding tissue 300 to an opposing side (e.g., an interior side) of the surrounding tissue 300.

The extensions 120 can be expanded to prevent passage of the tabs 118 through the openings 304, 306. In particular, the composition body 102 and the tabs 118 can maintain a tension or compressive force on the anchoring material 302 against the surrounding tissue 300. The anchoring material 302 can thereby be secured by the composition 170 against the wall of the surrounding tissue 300.

In certain embodiments, one or more compositions 170 can be used to interlock the anchoring material 302 to the surrounding tissue 300. In certain embodiments, one or more compositions 170 can be used to retain the anchoring material 302 over an incision formed in the surrounding tissue 300. For example, the surrounding tissue 300 can include two opposing sets of openings 304 around an incision and the anchoring material 302 can include two opposing sets of openings 306 complementary to the openings 304 of the surrounding tissue 300. Two or more compositions 170 can be used as described above to anchor the anchoring material 302 over the incision. Thus, the incision in the surrounding tissue 300 can be covered by the anchoring material 302 without the use of sutures.

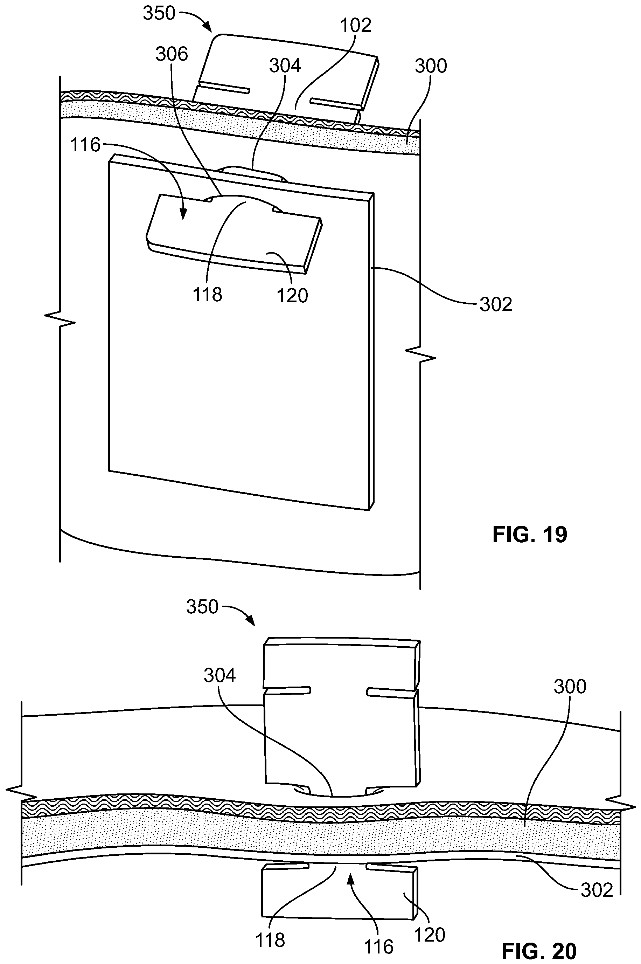

With reference to FIGS. 19 and 20, perspective and side cross-sectional views of an alternative embodiment of an exemplary composition 350 are provided. In particular, FIG. 19 shows a perspective, cross-sectional view of the composition 350 in an interlocked configuration with surrounding tissue 300, and FIG. 20 shows a side, cross-sectional view of the composition 350 in an interlocked configuration relative to the surrounding tissue 300. It should be understood that the composition 350 can be substantially similar in structure and function to the composition 170 discussed with respect to FIGS. 6 and 7, except for the distinctions noted herein.

Although illustrated with composition 350, it should be understood that in certain embodiments, composition 100 can be used in a substantially similar manner. Although illustrated as including an anchoring material 302 positioned adjacent to the surrounding tissue 300, it should be understood that in certain embodiments, additional materials can be positioned between, e.g., the anchoring material 302 and the surrounding tissue 300, the surrounding tissue 300 and the composition 350, the anchoring material 302 and the composition 350, combinations thereof, or the like.

In particular, rather than including multiple tabs 118 extending therefrom, the composition 350 can include only one pair of opposing tabs 118. The surrounding tissue 300 and the anchoring material 302 can include a single opening 304, 306, respectively, formed therein and passing therethrough. The openings 304, 306 can be complementary to the tab 118 of the composition 350. In particular, the openings 304, 306 can be configured and dimensioned to at least partially receive therethrough a tab 118 as shown in FIGS. 19 and 20 using the methods described above. The anchoring material 302 can thereby be secured by the composition 350 against the wall of the surrounding tissue 300.

In certain embodiments, one or more compositions 350 can be used to interlock the anchoring material 302 to the surrounding tissue 300. For example, the surrounding tissue 300 and the anchoring material 302 can include multiple complementary openings 304, 306 configured to receive individual compositions 350. In certain embodiments, one or more compositions 350 can be used to retain the anchoring material 302 over an incision formed in the surrounding tissue 300. For example, the surrounding tissue 300 can include a pair of two opposing openings 304 around an incision and the anchoring material 302 can include a pair of two opposing openings 306 complementary to the openings 304 of the surrounding tissue 300. Two or more compositions 350 can be used as described above to anchor the anchoring material 302 over the incision. Thus, the incision in the surrounding tissue 300 can be covered by the anchoring material 302 without the use of sutures.

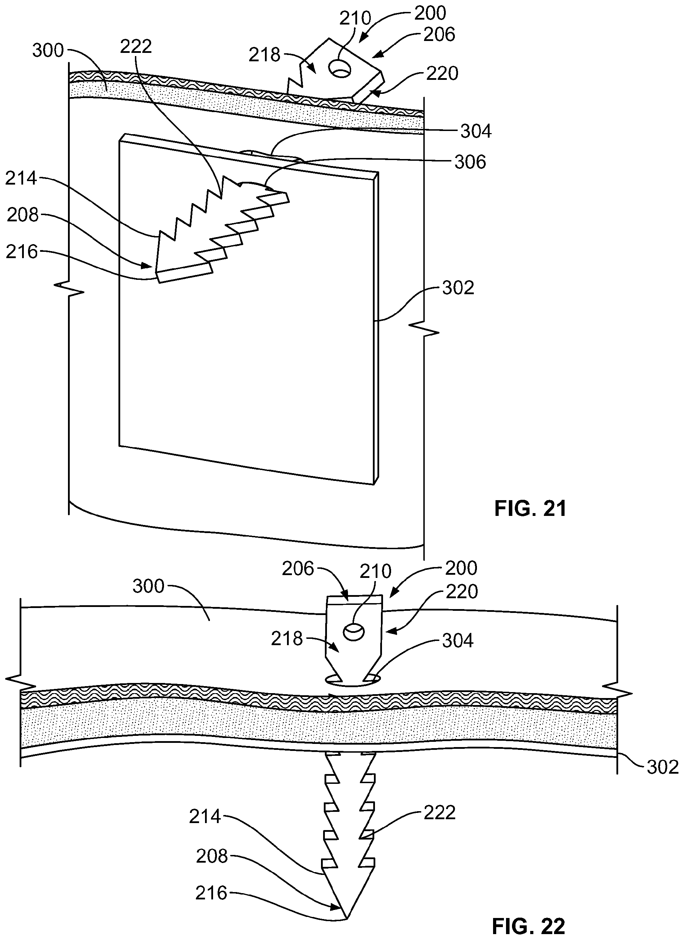

With reference to FIGS. 21 and 22, perspective and side cross-sectional views of an alternative embodiment of an exemplary composition 200 are provided. In particular, FIG. 21 shows a perspective, cross-sectional view of the composition 200 in an interlocked configuration with surrounding tissue 300, and FIG. 22 shows a side, cross-sectional view of the composition 200 in an interlocked configuration relative to the surrounding tissue 300. Although illustrated with composition 200, it should be understood that in certain embodiments, composition 250 can be used in a substantially similar manner.

In particular, rather than interlocking the composition 200 to itself or only to the surrounding tissue, in certain embodiments (similar to those described with respect to FIGS. 17-20), the composition 200 can be interlocked relative to an anchoring material 302, e.g., a separate piece of material. The anchoring material 302 can act as a fixation material or a bolt to maintain the composition 200 secured to the surrounding issue 300. Although illustrated as including an anchoring material 302 positioned adjacent to the surrounding tissue 300, it should be understood that in certain embodiments, additional materials can be positioned between, e.g., the anchoring material 302 and the surrounding tissue 300, the surrounding tissue 300 and the composition 200, the anchoring material 302 and the composition 200, combinations thereof, or the like.

As an example, the surrounding tissue 300 can include one or more openings 304 formed therein and passing therethrough. The openings 304 can be complementary to the second components 214, e.g., flared barbs, of the composition 200. In particular, each of the openings 304 can be configured and dimensioned to at least partially receive therethrough one or more of the second components 214 as shown in FIGS. 21 and 22.

The anchoring material 302 can also include one or more openings 306 formed therein and passing therethrough. The anchoring material 302 can be positioned on an opposing side of the surrounding tissue 300 relative to the composition 200 and the openings 306 of the anchoring material 302 can be aligned with the openings 304 of the surrounding tissue 300.

The distal end 208 and one or more second components 214 of the composition 200 can be passed through the respective openings 304, 306 until the anchoring material 302 is sufficiently anchored against the wall of the surrounding tissue 300. The non-angled portions of the second component 214 can prevent passage of the composition 200 back through the openings 304, 306, thereby maintaining the desired tension for anchoring the anchoring material 302 to the surrounding tissue 300.

In certain embodiments, rather than including a distal end having angled walls, the area surrounding the first component 210 can define substantially perpendicular side walls, e.g., a square or rectangular configuration. The perpendicular side wall positioned against the wall of the surrounding tissue 300 can therefore abut the surrounding tissue 300 and prevent further passage of the composition 200 through the opening 304. Thus, tension between the anchoring material 302 and the surrounding tissue 300 can be maintained by the composition 200.

In certain embodiments, one or more compositions 200 can be used to interlock the anchoring material 302 to the surrounding tissue 300. In certain embodiments, one or more compositions 200 can be used to retain the anchoring material 302 over an incision formed in the surrounding tissue 300. For example, the surrounding tissue 300 can include two or more opposing pairs of openings 304 around an incision and the anchoring material 302 can include two or more opposing pairs of openings 306 complementary to the openings 304 of the surrounding tissue 300. Two or more compositions 200 can be used as described above in the respective openings 304, 306 to anchor the anchoring material 302 over the incision. Thus, the incision in the surrounding tissue 300 can be covered by the anchoring material 302 without the use of sutures.

In certain embodiments, the compositions described herein can be wrapped at least partially around an implant (e.g., a breast implant, a pacemaker, or the like), tissue expanders, or both. For example, the compositions can be permanently or temporarily wrapped around the implant by affixing the composition to itself, the implant, or the surrounding tissue.

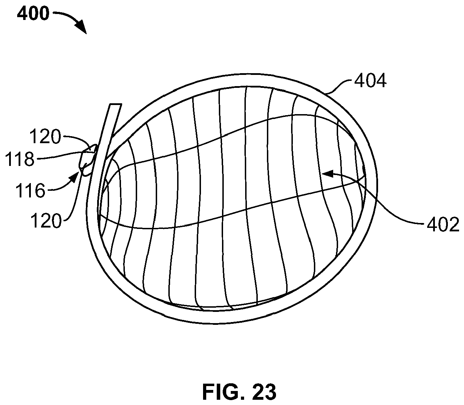

With reference to FIG. 23, a side view of a composition 400 wrapped around an implant 402 is provided. The structure and function of the composition 400 can be substantially similar in structure and function to the composition 100, except for the distinctions noted herein. Therefore, like structures are marked with like reference numbers.

The composition 400 can include the first and second components 114, 116 configured to interlock relative to each other. The width of the body 404 of the composition 400 can be dimensioned greater than the width 106 of the body 102 (see, e.g., FIG. 1). In particular, the body 404 can be wide enough to accommodate wrapping the composition around the implant 402 (e.g., a breast implant, a pacemaker, a tissue expander, or the like).

As illustrated in FIG. 23, the composition 400 can be wrapped tightly around the implant 402 and the first and second components 114, 116 can be interlocked to maintain the composition 400 in the wrapped configuration. In certain embodiments, rather than including first and second components 114, 116, the composition 400 can include two sets of opposing second components 116 (see, e.g., FIG. 6). Thus, instead of wrapping the composition 400 fully around the implant 402, the composition 400 can be partially wrapped around the implant 402 and the second components 116 can be interlocked with slots formed in surrounding tissue. The composition 400 can thereby be used to tether or secure the implant 402 to the surrounding tissue by forming a pocket between the composition 400 and the tissue configured and dimensioned to receive the implant 402 therein.

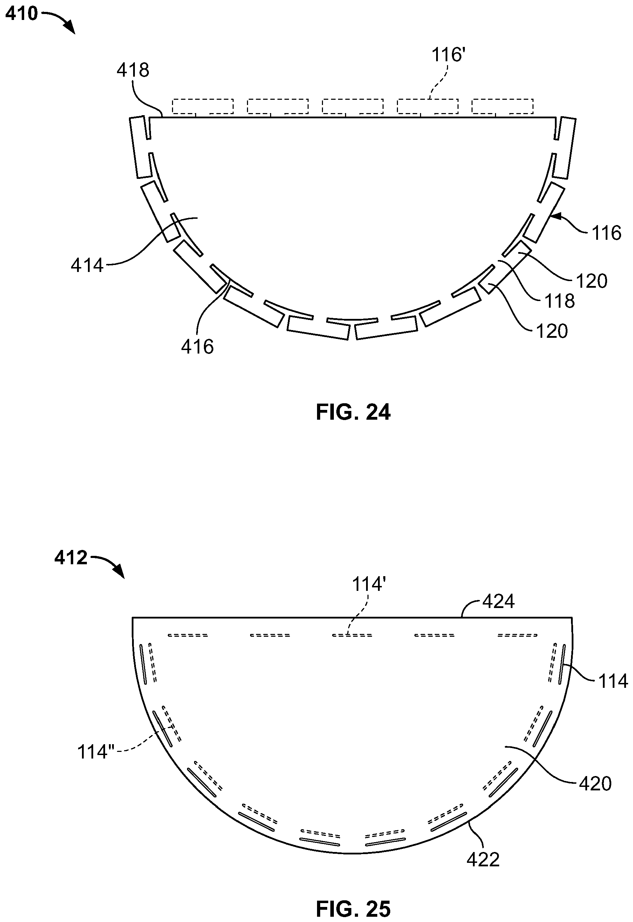

With reference to FIGS. 24 and 25, top views of a first portion 410 of a composition and a second portion 412 of a composition are provided. The structure and function of some of the components of the first and second portions 410, 412 can be substantially similar to the components of the composition 100, except for the distinctions noted herein. As such, like structures are marked with like reference numbers.

As will be described in greater detail below, the first and second portions 410, 412 can be configured and dimensioned to detachably interlock with each other to secure an implant 402 therebetween. The first portion 410 of the composition includes a composition body 414. The body 414 can be substantially semicircular in configuration. For example, the body 414 can define a substantially arcuate first side 416 and a substantially linear second side 418. In certain embodiments, the first portion 410 can define a substantially circular configuration without the linear second side 418.

The first side 416 can include a plurality of second components 116 extending therefrom. Each of the second components 116 can include a tab 118 and opposing extensions 120. In some embodiments, the outer edge of the second components 116 can define an arcuate shape complementary to the arcuate shape of the first side 416. In certain embodiments, as shown by the dashed lines, the second side 418 can include a plurality of second components 116' formed therein that are substantially similar in structure and function to the second components 116. Although illustrated as symmetrical in configuration, in certain embodiments, the configuration of the first portion 410 can be asymmetrical to conform to different shapes of implants 402.

The second portion 412 of the composition includes a composition body 420. The body 420 can be substantially semicircular in configuration and complementary to the body 414 of the first portion 410. In particular, the body 420 can define a substantially arcuate first side 422 and a substantially linear second side 424. In certain embodiments, the second portion 412 can define a substantially circular configuration without the linear second side 424.

The second portion 412 can include a plurality of first components 114 (e.g., a series of holes, slits, or the like) formed near and offset from the edge of the first side 422. In some embodiments, the first components 114 can define an arcuate shape complementary to the arcuate shape of the first side 422. The number and configuration of the first components 114 can be complementary to the second components 116 on the first portion 410 such that the first and second components 114, 114 can be detachably interlocked relative to each other.

In certain embodiments, the second side 424 of the second portion 412 can include a plurality of first components 114' formed therein that are substantially similar in structure and function to the first components 114. In particular, the first components 114' can be configured to interlock with the second components 116' of the first portion 410. In certain embodiments, the second portion 412 can include two or more sets of first components (e.g., first components 114, 114") to allow for customization of interlocking of the first and second portions 410, 412 relative to each other, thereby accommodating implants 402 of different dimensions.

Although illustrated as symmetrical in configuration, in certain embodiments, the configuration of the second portion 412 can be asymmetrical to conform to different shapes of implants 402. It should be understood that the configuration of the first and second portions 410, 412 can be complementary relative to each other to allow interlocking of the first and second portions 410, 412.

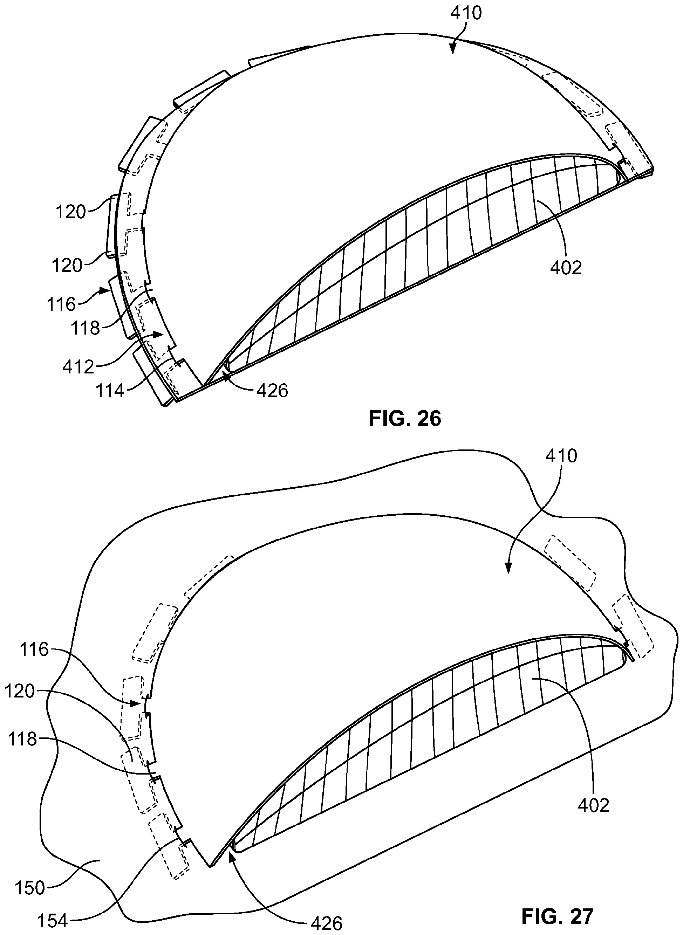

FIG. 26 is a perspective view of the first and second portions 410, 412 of a composition in an interlocked configuration and surrounding an implant 402. The implant 402 can be positioned between the first and second portions 410, 412, and the first and second components 114, 116 of the first and second portions 410, 412 can be interlocked relative to each other to tightly wrap around at least a portion of the implant 402. In particular, interlocking the first and second portions 410, 412 relative to each other forms a cavity 426 (e.g., a pocket) between the first and second portions 410, 412 configured and dimensioned to receive therein the implant 412. In certain embodiments, different sets of the first components 114" of the second portion 412 can be used to customize the dimensions of the cavity 426 formed between the first and second portions 410, 412, thereby accommodating implants 402 of different dimensions. Although illustrated as forming a cavity 426 with one end open, in certain embodiments, the first and second components 114, 114', 116, 116' can be used to completely encase the implant 402 between the first and second portions 410, 412.

FIG. 27 is a perspective view of the first portion 410 of a composition in an interlocked configuration with surrounding tissue 150 and surrounding an implant 402. In particular, rather than interlocking the first and second portions 410, 412, a plurality of incisions 154 (e.g., openings, slits, or the like) can be formed in the surrounding tissue 150 configured and dimensioned to interlock with the second components 116 of the first portion 410. The implant 402 can be positioned on the surrounding tissue 150 and the first portion 410 can be interlocked with the surrounding tissue 150 to tether the implant 402 to the surrounding tissue 150 within the cavity 426. Although illustrated as forming a cavity 426 with one end open, in certain embodiments, the second components 116, 116' can be used to completely encase the implant 402 between the first portion 410 and the surrounding tissue 150.

The compositions described herein can therefore provide suture-free wrapping and/or binding of one or more tissues, tendons, nerves, bones, implants, tissue expanders, combinations thereof, and the like, while having sufficient strength to prevent material failure of the compositions during application of force. In addition, the suture-free application of the compositions allows wrapping and/or binding in a time efficient manner.

Although the compositions and methods of the present disclosure have been described with reference to exemplary embodiments thereof, the present disclosure is not limited to such exemplary embodiments and or implementations. Rather, the compositions and methods of the present disclosure are susceptible to many implementations and applications, as will be readily apparent to persons skilled in the art from the disclosure hereof. The present disclosure expressly encompasses such modifications, enhancements and or variations of the disclosed embodiments. Since many changes could be made in the above exemplary embodiments and many widely different embodiments of this disclosure could be made without departing from the scope thereof, it is intended that all matter contained in the drawings and specification shall be interpreted as illustrative and not in a limiting sense. Additional modifications, changes, and substitutions are intended in the foregoing disclosure.

## Claims

1. A product for tissue treatment, comprising:
a body having an elongate length and a width, and including a centerline extending the length of the body and separating the body into a first half and a second half;
at least a first component formed in the first half of the body, the first component comprising one or more holes or slits passing through the body on one side of the centerline; and
at least a second component formed in the second half of the body, the second component comprising one or more tabs on an opposing side of the centerline, the one or more tabs being opposed to and aligned with the one or more holes or slits;
wherein the tab has two opposing extensions,
wherein the product is configured to be at least partially wrapped around or positioned onto tissue or an implant,
wherein the product comprises a tissue matrix, and
wherein the first and second components are complementary relative to each other and are configured to interlock relative to each other or to the tissue to maintain the product at least partially wrapped around or positioned on the tissue or the implant.

2. The product of claim 1, wherein the tissue matrix is an acellular tissue matrix.

3. The product of claim 1 or 2, wherein the tissue matrix is a dermal tissue matrix.

4. The product of any of claims 1-3, wherein the body is flexible to permit at least partially wrapping the body around the tissue or the implant, and interlocking of the first and second components relative to each other.

5. The product of any of claims 1-4, wherein the tab includes two opposing extensions along a length of the body, wherein the two opposing extensions are configured to be bent into a non-extending configuration to pass the two opposing extensions through the hole or slit in the body in a first direction, and wherein the two opposing extensions are configured to be unbent after passage of the two opposing extensions through the hole or slit in the body to prevent passage of the two opposing extensions through the hole or slit in the body in a second direction opposing the first direction.

6. The product of any of claims 1-5, wherein the first and second components are configured to remain interlocked relative to each other or the tissue without the use of suture.

7. The product of any of claims 1-6, wherein the centerline separates the body into a first half and a second half, the first and second halves dimensioned equal relative to each other.

8. The product of any of claims 1-7, wherein the holes or slits passing through the body extend parallel to the centerline.

9. The product of any of claims 1-8, wherein the one or more tabs extend perpendicularly relative to the centerline.

10. The product of any of claims 1-9, wherein the one or more tabs includes a proximal end and a distal end.

11. The product of any of claims 1-10, wherein the two opposing extensions of the one or more tabs extend parallel to the centerline.

## Patentansprüche

1. Produkt für die Gewebebehandlung, umfassend:
einen Körper, der eine langgestreckte Länge und eine Breite aufweist und eine Mittellinie umfasst, die sich über die Länge des Körpers erstreckt und den Körper in eine erste Hälfte und eine zweite Hälfte teilt;
mindestens eine erste Komponente, die in der ersten Hälfte des Körpers ausgebildet ist, wobei die erste Komponente ein oder mehrere Löcher oder Schlitze umfasst, die auf einer Seite der Mittellinie durch den Körper führen; und
mindestens eine zweite Komponente, die in der zweiten Hälfte des Körpers ausgebildet ist, wobei die zweite Komponente ein oder mehrere Laschen auf einer entgegengesetzten Seite der Mittellinie umfasst, wobei die eine oder mehreren Laschen dem einen oder den mehreren Löchern oder Schlitzen entgegengesetzt und an diesen ausgerichtet sind;
wobei die Lasche zwei entgegengesetzte Fortsätze aufweist,
wobei das Produkt so konfiguriert ist, dass es zumindest teilweise um Gewebe oder ein Implantat gewickelt oder auf diese aufgelegt werden kann,
wobei das Produkt eine Gewebematrix umfasst, und
wobei die erste und die zweite Komponente einander ergänzen und so konfiguriert sind, dass sie miteinander oder mit dem Gewebe verschränkt werden, um das Produkt zumindest teilweise um das Gewebe oder das Implantat gewickelt oder auf diese aufgelegt zu halten.

2. Produkt nach Anspruch 1, wobei die Gewebematrix eine azelluläre Gewebematrix ist.

3. Produkt nach Anspruch 1 oder 2, wobei die Gewebematrix eine Hautgewebematrix ist.

4. Produkt nach einem der Ansprüche 1-3, wobei der Körper flexibel ist, um zu ermöglichen, dass der Körper zumindest teilweise um das Gewebe oder das Implantat gewickelt wird und die erste und zweite Komponente miteinander verschränkt werden.

5. Produkt nach einem der Ansprüche 1-4, wobei die Lasche zwei entgegengesetzte Fortsätze entlang einer Länge des Körpers umfassen, wobei die beiden entgegengesetzten Fortsätze so konfiguriert sind, dass sie in eine sich nicht erstreckende Konfiguration gebogen werden, um die beiden entgegengesetzten Fortsätze in einer ersten Richtung durch das Loch oder den Schlitz im Körper zu führen, und wobei die beiden entgegengesetzten Fortsätze so konfiguriert sind, das sie nach dem Führen der beiden entgegengesetzten Fortsätze durch das Loch oder den Schlitz im Körper ausgestreckt werden, um das Führen der beiden entgegengesetzten Fortsätze durch das Loch oder den Schlitz im Körper in einer zweiten Richtung zu verhindern, die der ersten Richtung entgegengesetzt ist.

6. Produkt nach einem der Ansprüche 1-5, wobei die erste und die zweite Komponente so konfiguriert sind, dass die miteinander oder mit dem Gewebe ohne die Verwendung einer Naht verschränkt bleiben.

7. Produkt nach einem der Ansprüche 1-6, wobei die Mittellinie den Körper in eine erste Hälfte und eine zweite Hälfte teilt, wobei die Dimensionen der ersten und der zweiten Hälfte in Bezug zueinander gleich sind.

8. Produkt nach einem der Ansprüche1-7, wobei sich die Löcher oder Schlitze, die durch den Körper führen, parallel zur Mittellinie erstrecken.

9. Produkt nach einem der Ansprüche 1-8, wobei sich die eine oder mehreren Laschen bezüglich der Mittelline rechtwinklig erstrecken.

10. Produkt nach einem der Ansprüche1-9, wobei die eine oder mehreren Laschen ein proximales Ende und ein distales Ende umfassen.

11. Produkt nach einem der Ansprüche 1-10, wobei sich die beiden entgegengesetzten Fortsätze der einen oder mehreren Laschen parallel zur Mittellinie erstrecken.

## Revendications

1. Produit de traitement de tissu, comprenant :
un corps ayant une longueur allongée et une largeur, et comprenant une ligne centrale s'étendant sur la longueur du corps et séparant le corps en une première moitié et une seconde moitié ;
au moins un premier composant formé dans la première moitié du corps, le premier composant comprenant un ou plusieurs trous ou fentes passant à travers le corps sur un côté de la ligne centrale ; et
au moins un second composant formé dans la seconde moitié du corps, le second composant comprenant une ou plusieurs languettes sur un côté opposé de la ligne centrale, la ou les languettes étant opposées à et alignées avec le ou les trous ou fentes ;
la languette ayant deux extensions opposées,
le produit étant configuré pour être au moins partiellement enveloppé autour ou positionné sur un tissu ou un implant,
le produit comprenant une matrice tissulaire, et
les premier et second composants étant complémentaires l'un par rapport à l'autre et étant configurés pour se verrouiller l'un par rapport à l'autre ou au tissu pour maintenir le produit au moins partiellement enveloppé autour ou positionné sur le tissu ou l'implant.

2. Produit selon la revendication 1, dans lequel la matrice tissulaire est une matrice tissulaire acellulaire.

3. Produit selon la revendication 1 ou 2, dans lequel la matrice tissulaire est une matrice tissulaire dermique.

4. Produit selon l'une quelconque des revendications 1 à 3, dans lequel le corps est souple pour permettre d'envelopper au moins partiellement le corps autour du tissu ou de l'implant, et de verrouiller les premier et second composants l'un par rapport à l'autre.

5. Produit selon l'une quelconque des revendications 1 à 4, dans lequel la languette comprend deux extensions opposées le long d'une longueur du corps, les deux extensions opposées étant configurées pour être courbées dans une configuration non étendue pour faire passer les deux extensions opposées à travers le trou ou la fente dans le corps dans une première direction,
et les deux extensions opposées étant configurées pour être non courbées après le passage des deux extensions opposées à travers le trou ou la fente dans le corps afin d'empêcher le passage des deux extensions opposées à travers le trou ou la fente dans le corps dans une seconde direction opposée à la première direction.

6. Produit selon l'une quelconque des revendications 1 à 5, dans lequel les premier et second composants sont configurés pour rester verrouillés l'un par rapport à l'autre ou au tissu sans utiliser de suture.

7. Produit selon l'une quelconque des revendications 1 à 6, dans lequel la ligne centrale sépare le corps en une première moitié et une seconde moitié, les première et seconde moitiés étant dimensionnées égales l'une par rapport à l'autre.

8. Produit selon l'une quelconque des revendications 1 à 7, dans lequel les trous ou les fentes passant à travers le corps s'étendent parallèlement à la ligne centrale.

9. Produit selon l'une quelconque des revendications 1 à 8, dans lequel la ou les languettes s'étendent perpendiculairement à la ligne centrale.

10. Produit selon l'une quelconque des revendications 1 à 9, dans lequel la ou les languettes comprennent une extrémité proximale et une extrémité distale.

11. Produit selon l'une quelconque des revendications 1 à 10, dans lequel les deux extensions opposées de la ou des languettes s'étendent parallèlement à la ligne centrale.
